# EUROPÄISCHE PATENTSCHRIFT

(11) **EP 1 427 698 B1**
(45) Veröffentlichungstag und Bekanntmachung des Hinweises auf die Patenterteilung: **02.12.2009**
(21) Anmeldenummer: 02767438.1
(22) Anmeldetag: 27.08.2002
(51) Int. Cl.: C07C 309/06, H01M 6/16, H01M 10/40

(54) **HEPTAFLUOR-2-PROPANSULFONATSALZE UND IHRE VERWENDUNG IN DER ELEKTROTECHNIK**
HEPTAFLUORO-2-PROPANE SULFONATE SALTS AND THE USE THEREOF IN ELECTRONIC TECHNOLOGY
SELS D'HEPTAFLUORO-2-PROPANE-SULFONATE ET LEUR UTILISATION EN ELECTROTECHNIQUE

(30) Priorität: 04.09.2001 DE 10143172
(43) Veröffentlichungstag der Anmeldung: 16.06.2004
(73) Patentinhaber: Solvay Fluor GmbH, 30173 Hannover (DE)
(72) Erfinder: BÖSE, Olaf, 30167 Hannover (DE); FRANKLIN, James, B-1170 Brussels (BE); PETERKORD, Katja, 30175 Hannover (DE)
(74) Vertreter: Jacques, Philippe
(86) Internationale Anmeldenummer: PCT/EP2002/009544
(87) Internationale Veröffentlichungsnummer: WO 2003/020691

(56) Entgegenhaltungen:
- DE-A- 3 913 037
- DATABASE CA [Online] CHEMICAL ABSTRACTS SERVICE, COLUMBUS, OHIO, US; MORIMOTO, TAKESHI ET AL: "Electric double-layer capacitor" retrieved from STN Database accession no. 107:167115 CA XP002226286 & JP 62 090919 A (ASAHI GLASS CO., LTD., JAPAN;ELNA CO., LTD.) 25. April 1987 (1987-04-25)
- DATABASE CA [Online] CHEMICAL ABSTRACTS SERVICE, COLUMBUS, OHIO, US; MORIMOTO, TAKESHI ET AL: "Electric double layer capacitors" retrieved from STN Database accession no. 106:225893 CA XP002226287 & JP 61 252620 A (ASAHI GLASS CO., LTD., JAPAN) 10. November 1986 (1986-11-10)
- PATENT ABSTRACTS OF JAPAN vol. 013, no. 515 (C-655), 17. November 1989 (1989-11-17) & JP 01 207263 A (SONY CORP), 21. August 1989 (1989-08-21)
- DATABASE CA [Online] CHEMICAL ABSTRACTS SERVICE, COLUMBUS, OHIO, US; YAMAOKA, SEIJI ET AL.: "Preparation of perfluoroalkylsulfonates" retrieved from STN Database accession no. 135:371444 XP002226288 & JP 2001 322975 A (DAINIPPON INK AND CHEMICALS ) 20. November 2001 (2001-11-20)

## Beschreibung

Die Erfindung bezieht sich auf neue Salze von Hepta-fluor-2-Propansulfonat und ihre Verwendung in der Elektrotechnik.

Alkalimetall- und Tetraalkylammoniumsalze von bestimmten Anionen sind in der Elektrotechnik brauchbar. Tetraalkylammoniumsalze werden z. B. in Doppelschicht-Kondensatoren eingesetzt. Alkalimetallsalze, insbesondere das Lithiumsalz des Hexafluorphosphat-Anions, des Tetrafluorborat-Anions, des Trifluormethansulfonat-Anions oder des Bis-Trifluormethansulfonylimid, werden als Leitsalz für Lithiumionen-Batterien eingesetzt. Das jeweils verwendete Leitsalz wird zu diesem Zweck in einem Elektrolytlösungsmittel gelöst. Gut geeignete Elektrolytlösemittel sind beispielsweise Alkylencarbonate, Dialkylcarbonate, Ether, Formamide, Sulfolane oder Methylsulfolane sowie Nitrile, andere stickstoffhaltige Verbindungen wie Nitromethan oder Pyrrolidinone. Solche Verbindungen werden von Makoto Ue, Kazuhiko Ida und Shoichiro Mori in J. Electrochem. Soc. 141(1994), Seiten 2989 bis 2996 zusammengestellt. Die Verwendbarkeit von fluorierten Phosphorsäureestern, z. B. von Tris(trifluorethyl)phosphat, wird in der EP-A 825 664 offenbart, von Estern von Phosphon- oder Phosphinsäuren in der US-A 6,210,840. Perfluoralkylsulfonsäuresalze als leitsalze werden in JP 62090919 und JP 61252620 offenbart.

Es wurde jetzt gefunden, daß bestimmte Alkalimetall- und Tetraalkylammoniumsalze sehr gut in der Elektrotechnik, beispielsweise als Leitsalz für Batterien oder in Kondensatoren, brauchbar sind.

'Mit der vorliegenden Erfindung werden Verbindungen der allgemeinen Formel (I) zur Verfügung gestellt,

(CF₃)₂CFSO₃⁻ M⁺ (I),

worin M⁺ für Li⁺, Na⁺, K⁺, Cs⁺, Rb⁺, R₄P⁺ oder R₄N⁺ steht, worin die 4 Alkylgruppen R gleich oder verschieden sein können und für C1-C4-Alkyl stehen oder mindestens 2 R-Gruppen den Stickstoff oder Phosphor einschließen und ein nichtaromatisches Ringsystem bilden.

Etwaig als Zwischenprodukt benötigtes Perfluorisopropylsulfonylfluorid (auch als Heptafluoro-2-propansulfonsäure-fluorid bezeichnet) kann aus einem entsprechenden Isopropansulfonylhalogenid durch Elektrolyse in Anwesenheit von wasserfreiem flüssigen Fluorwasserstoff hergestellt werden, siehe z. B. US-Patent 3,809,711.

Eine andere Herstellungsmethode wird von Stanley Temple in J. Org. Chem. 33(1968) auf den Seiten 344 bis 346 vorgestellt. Dabei wird Sulfurylfluorid an Hexafluorpropen angelagert. Als Katalysator wird Cesiumfluorid verwendet oder auch Tetraethylammoniumbromid.

Für die Herstellung der Alkalisalze der Verbindungen der Formel (I) bieten sich zwei sehr gute Methoden an. Bei der ersten Methode wird aus Heptafluoro-2-propansulfonylfluorid durch Umsetzung mit Calciumhydroxid zunächst das entsprechende Calciumsalz hergestellt. Das Calciumsalz wird dann mit Schwefelsäure in die freie Sulfonsäure umgewandelt. Die freie Sulfonsäure wird wiederum mit dem Alkalimetallhydroxid unter Bildung des Alkalimetallsalzes zur Reaktion gebracht. Die zweite Methode gestattet die direkte Herstellung des Alkalimetallsalzes durch Umsetzung von Heptafluoro-2-propansulfonylfluorid mit zwei Äquivalenten des Alkalimetallhydroxids.

Die Herstellung der Ammonium- und Phosphoniumsalze des Heptafluor-2-propansulfonats kann nach bekannten Synthesewegen erfolgen. Die Herstellung der Phosphoniumsalze kann beispielsweise gemäß der Publikation von Kang Xu, Michael S. Ding und T. Richard Jow aus Journal of The Electrochemical Soc., 148(3) A267-A274 (2001) durchgeführt werden. In Tabelle 1 dieser Veröffentlichung ist unter der Bezeichnung "Ue-Mori Process" ein Syntheseweg für Phosphoniumsalze angegeben. Dabei wird ein Trialkylphosphin mit einem Dialkylcarbonat in Methanol bei 100 °C umgesetzt. Je nach Auswahl des Dialkylcarbonats können Phosphoniumsalze erzeugt werden, in welchen alle vier Substituenten R gleich sind, oder in welchen mindestens ein Substituent R eine andere Bedeutung hat als die anderen drei Substituenten. Bei der Umsetzung des Phosphins mit dem Dialkylcarbonat entsteht zunächst das Tetraalkylphosphoniumalkylcarbonat, welches bei Zusatz der Heptafluoro-2-propansulfonsäure unter Bildung des gewünschten Salzes reagiert. Diese Methode entspricht dem bereits zitierten "Ue-Mori Process" in der Publikation von Kang Xu und Mitarbeitern.

Andere Methoden zur Herstellung der Tetraalkylammoniumsalze sehen vor, zunächst in einer sogenannten "Menschutkin-Reaktion" aus einem Trialkylamin und einem Alkylhalogenid das quaternäre Tetraalkylammoniumhalogenid-Salz herzustellen.

Das auf diese Weise erhaltene Halogenidsalz, beispielsweise das Bromid oder Chlorid, kann auf verschiedenen Wegen zum gewünschten Produkt umgewandelt werden. Das Tetraalkylammoniumhalogenid kann beispielsweise mit einem Alkalisalz, beispielsweise dem Natriumsalz, der Heptafluor-2-propansulfonsäure unter Membrandialyse zur Abtrennung des gebildeten Alkalimetallhalogenids umgesetzt werden. Eine andere Methode sieht vor, das Tetraalkylammoniumhalogenid mit einem Alkalimetallsalz, beispielsweise dem Natriumsalz, der Heptafluor-2-propansulfonsäure unter kontinuierlicher Extraktion des gebildeten Produktes durch beispielsweise Dichlormethan umzusetzen. Das Alkalihalogenid, beispielsweise in Form von Natriumbromid, verbleibt in der wäßrigen Phase.

Eine weitere Methode sieht vor, daß man das Tetraalkylammoniumhalogenid zunächst in das Tetraalkylammoniumhydroxid umwandelt. Dies ist möglich durch Zugabe von beispielsweise KOH und Methanol; möglich ist es auch durch Elektrolyse oder durch Ionenaustauscherharze. Das erhaltene Tetraalkylammoniumhydroxid wird dann mit der Heptafluor-2-propansulfonsäure unter Bildung des gewünschten Tetraalkylammoniumsalzes dieser Säure umgesetzt.

Die Herstellung von Ammoniumkationen mit drei unterschiedlichen Arten des Substituenten R wird in der Publikation von Kang Xu und seinen Mitarbeitern in Tabelle 1 als Methode "Eschweiler-Clarke and Ue-Mori Process" erläutert. Ein Dialkylamin wird zunächst mit Acetaldehyd in Ameisensäure zum Dialkylethylamin umgesetzt und dann mit einem Dialkylcarbonat quaternisiert.

In analoger Weise können auch Ammoniumkationen hergestellt werden, bei denen mindestens zwei der Gruppen R einen Ring bilden, der das Stickstoffatom enthält. Diese Ringsysteme haben vorzugsweise fünf, sechs oder sieben Atome im Ring.

Einige Tetraalkylammoniumkationen sind in Form der Hydroxide ein Handelsprodukt. Sie können dann mit der Hepta-fluor-2-propansulfonsäure zum gewünschten Salz umgesetzt werden.

Bevorzugt besitzt M⁺ die Bedeutung Li⁺ oder R₄N⁺. Im Tetraalkylammoniumkation besitzt der Substituent R vorzugsweise die Bedeutung Methyl oder Ethyl; dabei können die vier Substituenten R gleich oder verschieden sein. Beispiele für solche Kationen sind Me₄N⁺, Me₃EtN⁺, n-Pr₃MeN⁺, i-Pr₂EtMeN⁺, n-Bu₃MeN⁺, Me₄P⁺, Et₄P⁺, Et₄N⁺, Et₃MeP⁺, n-Bu₃MeP⁺ sowie N,N-Dimethylpiperidinium.

Die zur Verfügung gestellten Verbindungen der allgemeinen Formel (I) eignen sich sehr gut zur Anwendung in der Elektrotechnik, beispielsweise als Leitsalz gelöst in einem Lösemittel (Elektrolyt). Das Tetraalkylammoniumsalz und das Tetraalkylphosphoniumsalz des Heptafluorpropansulfonats kann beispielsweise in Kondensatoren eingesetzt werden. Dabei handelt es sich um Doppelschichtkondensatoren, die einen elektrochemischen Energiespeicher darstellen. Die elektrische Ladung wird in der elektrischen Doppelschicht gespeichert, die zwischen einer polarisierbaren Elektrode und einer Elektrolytlösung entsteht, wenn eine Gleichspannung angelegt wird. Derartige Kondensatoren werden als "Memory back up"-Einrichtung in vielen elektronischen Geräten eingesetzt und wo große kurze Stromstöße hoher Stromstärken ("High Pulse Power") benötigt werden, z. B. in Elektroautos. Brauchbare Lösungsmittel sind in der zitierten Literaturstelle von Makoto Ue und Mitarbeitern angegeben.

Die Alkalimetallsalze, insbesondere das Lithiumkation, eignen sich besonders als Leitsalz für wiederaufladbare Batterien, besonders Lithiumionen-Batterien. Im Hinblick auf diese bevorzugte Anwendung der bevorzugten Lithiumsalze wird die Erfindung weiter erläutert.

Lithiumionen-Batterien enthalten das Leitsalz gelöst in einem Lösungsmittel oder einem Lösungsmittelgemisch. Die in der vorgenannten Veröffentlichung von Ue und Mitarbeitern genannten Lösungsmittel sind in reiner oder gemischter Form auch für den vorliegenden Anwendungsbereich bei Lithiumionen-Batterien brauchbar. Besonders gut brauchbar sind beispielsweise Dialkylcarbonate, Alkylencarbonate, Lactone und Formamide. Fluorsubstituierte organische Verbindungen sind ebenfalls als Lösungsmittel oder als Lösungsmittelzusatz brauchbar; ihr Vorteil ist, daß sie eine flammhemmende Wirkung aufweisen. Gut brauchbar sind z. B. die fluorhaltigen Ether, die in der EP-A 0 807 986 beschrieben sind, die teilfluorierten Carbonate der EP-A 0 887 874, die Dioxolone der WO 01/38319, die Amidine perfluorierter C2-C5-Carbonsäuren der nicht vorveröffentlichten deutschen Anmeldung ...(100 04 978.1), die fluorierten Alkylcarbonate der nicht vorveröffentlichten deutschen Anmeldung ... (101 13 902.4), die Difluormalonester der nicht vorveröffentlichten deutschen Patentanmeldung ...(100 37 627.4), und die, in der WO 00/38264 offenbarten, alkylenverbrückten Diester. Sehr vorteilhaft sind auch Dialkylamide von fluorierten Carbonsäuren, beispielsweise von N,N-Dialkylamiden der Trifluoressigsäure, die z. B. in der WO-00/38259 offenbart sind. "Alkyl" steht hier vorzugsweise für C1-C4-Alkyl. Sehr gut geeignet sind auch fluorsubstituierte Phosphor-, Phosphon- und Phosphinsäureester, die weiter oben schon erwähnt wurden und z. B. in der EP-A 825 664 bzw. der US-A 6,210,840 erwähnt werden.

Neben der Verwendung der Verbindungen der allgemeinen Formel (I) in der Elektrotechnik, insbesondere als Leitsalz in Batterien und Kondensatoren, sind Elektrolyte, die eine Verbindung der allgemeinen Formel (I) sowie ein Elektrolytlösemittel oder ein Elektrolytlösemittelgemisch enthalten, ebenfalls Gegenstand der Erfindung. Elektrolyte, die eine Verbindung der allgemeine Formel (I) enthalten, in welcher M⁺ für das Natrium-, Kalium-, Cesium-, Rubidium- oder Lithiumion steht, werden bevorzugt für Batterien, insbesondere Lithiumionen-Batterien eingesetzt; erfindungsgemäße Elektrolyte mit Verbindungen der Formel (I), in welchen M⁺ für das Tetraalkylammoniumion oder Tetraalkylphosphoniumion steht, werden bevorzugt für Kondensatoren eingesetzt. Die bevorzugten Salze und die bevorzugten Lösemittel bzw. Lösemittelgemische sind bereits weiter oben genannt. Ein Gegenstand der vorliegenden Erfindung sind auch Batterien, vorzugsweise Lithiumionen-Batterien, die eine Verbindung der allgemeinen Formel (I) enthalten. Auch hier gilt bezüglich der bevorzugten Salze und Lösemittel das oben Gesagte.

Schließlich sind auch Kondensatoren, die eine Verbindung der allgemeinen Formel (I) enthalten, insbesondere Kondensatoren mit einer elektrischen Doppelschicht, wie sie beispielsweise in der obengenannten Publikation von Ue und Mitarbeitern beschrieben werden, Gegenstand der Erfindung. Bevorzugte Elektrolyte (gelöste Salze und Lösemittel) sind oben schon genannt.

Vorteil der erfindungsgemäßen Verbindungen der allgemeinen Formel (I) ist, ihre hohe hydrolytische und thermische Stabilität z. B. im Vergleich mit dem häufig eingesetzten LiPF₆.

Die folgenden Beispiele sollen die Erfindung weiter erläutern, ohne sie in ihrem Umfang einzuschränken.

### Beispiel 1:

### Herstellung von Lithiumheptafluoro-2-propansulfonat, (CF₃)₂CFSO₃Li

Methode 1: Über das Ca-Salz und die freie Säure

a) 2(CF₃)₂CFSO₂F + 2Ca(OH)₂ ⇒[(CF₃)₂CFSO₃]₂Ca+CaF₂+₂H₂O

Heptafluoro-2-propansulfonylfluorid (50,4 g; 0,2 mol; 252 g mol⁻¹) wurde in einem geschlossenem Reaktionsgefäß mit einer Aufschlämmung von Calciumhydroxid (16,3 g; 0,22 mol; 74 g mol⁻¹) in 50 ml Wasser 8 h bei Raumtemperatur gerührt. Die wäßrige Lösung wurde vom entstandenen CaF₂ abfiltriert und zur Trockene eingeengt. Die Ausbeute an [(CF₃)₂CFSO₃]₂Ca (62,4 g; 0,11 mol; 538 g mol⁻¹) betrug 58,0 %.
*[(CF₃)₂CFSO₃]₂Ca in D6-Aceton*

| | |
|---|---|
| ¹³C-NMR | 95,55 ppm (CF, Dublett von Heptaletts); 120,55 ppm (CF₃; Dublett von Quartetts) |
| ¹⁹F-NMR | -73, 47 ppm (CF₃, Dublett) ; -172,87 ppm (CF, Heptalett) |

b) [(CF₃)₂CFSO₃]₂CA + H₂SO₄ ⇒ 2(CF₃)₂CFSO₃H + Ca₂SO₄

Getrocknetes [(CF₃)₂CFSO₃]₂Ca (53,8 g; 0,1 mol; 538 g mol⁻¹) aus der Stufe a) wurde mit einem Überschuß von 100 % H₂SO₄ umgesetzt. Die freie Sulfonsäure (CF₃)₂CFSO₃H wurde direkt aus dem Reaktionsansatz im Vakuum abdestilliert. Die Ausbeute an (CF₃)₂CFSO₃H (15,7 g; 0,0628 mol; 250 g mol⁻¹) betrug 62,8 %.
*(CF₃)₂CFSO₃H in D₂O*

| | | |
|---|---|---|
| ¹³C-NMR | 93,68 ppm | (CF, Dublett von Heptaletts); 118,62 ppm |
| | | (CF₃; Dublett von Quartetts) |
| ¹⁹F-NMR | -74,22 ppm | (CF₃, Dublett); -173,08 ppm |
| | | (CF, Heptalett) |

c) (CF₃)₂CFSO₃H + LiOH ⇒ (CF₃)₂CFSO₃Li + H₂O

(CF₃)₂CFSO₃H (10,0 g; 0,04 mol; 250 g mol⁻¹) aus Stufe b) wurde in wäßriger Lösung mit LiOH (0,96 g; 0,04 mol; 24 g mol⁻¹) neutralisiert und zur Trockene eingeengt. Die Ausbeute an (CF₃)₂CFSO₃Li (9,7 g; 0,038 mol; 256 g mol⁻¹) betrug 95,0 %.
*(CF₃)₂CFSO₃Li in D6-Aceton*

| | | |
|---|---|---|
| ¹³C-NMR | 95,55 ppm | (CF, Dublett von Heptaletts); 121,16 ppm |
| | | (CF₃; Dublett von Quartetts) |
| ¹⁹F-NMR | -73, 18 ppm | (CF₃, Dublett); -172, 78 ppm |
| | | (CF, Heptalett) |

Methode 2: Direkt aus dem Sulfonylfluorid und der Base

(CF₃)₂CFSO₂F + 2LiOH ⇒ (CF₃)₂CFSO₃Li + LF + H₂O

Heptafluoro-2-propansulfonylfluorid (25,2 g; 0,1 mol; 252 g mol⁻¹) wurde in einem geschlossenem Reaktionsgefäß mit einer Lösung von Lithiumhydroxid (4,8 g; 0,2 mol; 24 g mol⁻¹) in 30 ml Wasser 24 h bei Raumtemperatur gerührt. Die wäßrige Lösung wurde zur Trockene eingeengt. Der Rückstand wurde mit einem organischen Lösungsmittel, beispielsweise Dimethylcarbonat, extrahiert, um das entstandene (CF₃)₂CFSO₃Li vom LiF abzutrennen. Die organische Phase wurde getrocknet und anschließend eingeengt. Die Ausbeute an (CF₃)₂CFSO₃Li (20,7 g; 0,0808 mol; 256 g mol⁻¹) betrug 80,8 %. Das erhaltene Produkt war mit dem gemäß Methode 1 erhaltenen Produkt identisch.

### Beispiel 2:

### Elektrolyte mit Lithiumheptafluoro-2-Propansulfonat

Die Elektrolyte wurden durch Auflösen des Salzes im jeweiligen Lösemittelgemisch hergestellt. Dabei stellen die Werte von 2 Mol/l in Beispiel 2.2. bzw. 2.4. noch nicht die Grenzen für die Löslichkeit dar. Die verwendeten Lösungsmittel und die vorliegenden Salzkonzentrationen sind in Tabelle 1 zusammengestellt.

Dabei ist zu beachten, daß üblicherweise Salzkonzentrationen von 1 Mol/l in Lithiumionen-Batterien angewendet werden. Aus der hohen Löslichkeit des erfindungsgemäßen Lithiumsalzes kann geschlossen werden, daß Lithiumionen-Batterien mit dem Lithiumsalz auch bei tiefen Temperaturen verwendet werden können, ohne daß das Ausfallen des Salzes zu befürchten ist.

**Tabelle 1:**

| Elektrolyte mit Lithiumheptafluoro-2-Propansulfonat | | | |
|---|---|---|---|
| **Beispiel** | Salzkonzentration | Lösemittel (vol:vol)¹⁾ | |
| **2.1.** | 1 mol/Liter | EC:DEC | 1:1 |
| **2.2.** | 2 mol/Liter | EC:DEC | 1:1 |
| **2.3.** | 1 mol/Liter | EC:PC:DMC | 1:1:3 |
| **2.4.** | 2 mol/Liter | EC:PC:DMC | 1:1:3 |

| | | | |
|---|---|---|---|
| ¹⁾ EC = Ethylencarbonat PC = Propylencarbonat DEC = Diethylcarbonat DMC = Dimethylcarbonat | | | |

## Patentansprüche

1. Verbindungen der allgemeinen Formel 1
(CF₃)₂CFSO₃- M⁺ (I)
worin M⁺ für K⁺, Na⁺, Li⁺, Cs⁺, Rb⁺, R₄P⁺ oder RₐN⁺ steht, worin die 4 Alkylgruppen R gleich oder verschieden sein können und für C1-C4-Alkyl stehen oder mindestens 2 R-Gruppen den Stickstoff oder Phosphor einschließen und ein nichtaromatisches Ringsystem bilden.

2. Verbindungen nach Anspruch 1, worin M⁺ für Li⁺ oder R₄N⁺ stehen, worin die 4 Substituenten R gleich oder verschieden sein können und C1-C2-Alkyl bedeuten.

3. Verfahren zur Herstellung von Verbindungen der allgemeinen Formel (I),
A) wobei man zur Herstellung der Alkalimetallsalze
a) (CF₃)₂CFSO₂F mit dem Alkalihydroxid, vorzugsweise in wäßriger Lösung, umsetzt und das gebildete Alkalimetallheptailuorpropansulfonat vom gebildeten Alkalimetallfluorid abtrennt, oder
b) (CF₃)₂CFSO₂F mit Ca(OH)₂, vorzugsweise in wäßriger Lösung, umsetzt, das gebildete Calciumheptafluorpropansulfonat vom gefällten Calciumfluorid abtrennt, das Calciumheptafluorpropansulfonat mit Säure, vorzugsweise Schwefelsäure, in Heptafluorpropansulfonsäure und das Calciumsalz der Säure überführt und die Heptafluorpropansulfonsäure mit Alkalimetallhydroxid, vorzugsweise in wäßriger Lösung, unter Bildung des Alkalimetallheptafluorpropansulfonats umsetzt, oder
B) wobei man zur Herstellung der Tetraalkylammoniumsalze
a) Tetraalkylaminoniumhalogenid mit einem Alkalimetallsalz des Heptafluorpropansulfonats umsetzt und das gebildete Tetraalkylammoniumheptafluorpropansulfonat isoliert, oder
b) ein Tetraalkylammoniumhydroxid mit Heptafluorpropansulfonsäure umsetzt, oder
C) wobei man zur Herstellung der Tetraalkylammonium- oder Tetraalkylphosphoniumsalze ein Tetraalkylammonium- oder Tetraalkylphosphoniumalkylcarbonat mit Heptafluorpropansulfonsäure umsetzt.

4. Verwendung von Verbindungen der allgemeinen Formel (I) in der Elektrotechnik.

5. Verwendung nach Anspruch 4, wobei Verbindungen der allgemeinen Formel (I), worin M⁺ für Na⁺, K⁺, Cs⁺, Rb⁺, oder Li⁺ steht, als Leitsalz für Batterien.

6. Verwendung nach Anspruch 5, worin M⁺ für Li⁺ steht, als Leitsalz für Lithiumionen-Batterien.

7. Verwendung von Verbindungen der allgemeinen Formel (I), worin M⁺ für R₄P⁺ oder R₄N⁺ steht, als Leitsalz in Kondensatoren.

8. Elektrolyt, enthaltend eine oder mehrere Verbindungen der allgemeinen Formel (I) als Leitsalz und ein Elektrolytlösemittel.

9. Batterie, enthaltend einen Elektrolyt des Anspruchs 8.

10. Batterie, nach Anspruch 9, wobei M⁺ in der Formel (CF₃)₂CFSO₃⁻M⁺(I) Li⁺ bedeutet.

11. Kondensator, enthaltend eine Verbindung der allgemeinen Formel (I), worin M⁺ für R₄P⁺ oder für R₄N⁺ steht.

## Claims

1. Compounds of the general formula I
(CF₃)₂CFSO₃-M⁺ (I)
where M⁺ is K⁺, Na⁺, Li⁺, Cs⁺, Rb⁺, R₄P⁺ or R₄N⁺, where the 4 alkyl groups R can be identical or different and are each C1-C4-alkyl or at least 2 groups R include the nitrogen or phosphorus and form a nonaromatic ring system.

2. Compounds according to Claim 1 in which M⁺ is Li⁺ or R₄N⁺, where the 4 substituents R can be identical or different and are each C1-C2-alkyl.

3. Process for preparing compounds of the general formula (I), wherein
A) the alkali metal salts are prepared by
a) reacting (CF₃)₂CFSO₂F with the alkali metal hydroxide, preferably in aqueous solution, and separating off the alkali metal heptafluoropropanesulphonate formed from the alkali metal fluoride formed or
b) reacting(CF₃)₂CFSO₂F with Ca(OH)₂, preferably in aqueous solution, separating off the calcium heptafluoropropanesulphonate formed from the precipitated calcium fluoride, converting the calcium heptafluoropropanesulphonate by means of acid, preferably sulphuric acid, into heptafluoropropanesulphonic acid and the calcium salt of the acid and reacting the heptafluoropropanesulphonic acid with alkali metal hydroxide, preferably in aqueous solution, to form the alkali metal heptafluoropropanesulphonate, or
B) the tetraalkylammonium salts are prepared by
a) reacting tetraalkylammonium halide with an alkali metal salt of heptafluoropropanesulphonate and isolating the tetraalkylammonium heptafluoropropanesulfonate formed or
b) reacting a tetraalkylammonium hydroxide with heptafluoropropanesulphonic acid, or
C) the tetraalkylammonium or tetraalkylphosphonium salts are prepared by reacting a tetraalkylammonium or tetraalkylphosphonium alkylcarbonate with heptafluoropropanesulphonic acid.

4. Use of compounds of the general formula (I) in electronic technology.

5. Use according to Claim 4 of compounds of the general formula (I) in which M⁺ is Na⁺, K⁺, Cs⁺, Rb⁺ or Li⁺ as electrolyte salt for batteries.

6. Use according to Claim 5, wherein M⁺ is Li⁺, as electrolyte salt for lithium ion batteries.

7. Use of compounds of the general formula (I) in which M⁺ is R₄P⁺ or R₄N⁺ as electrolyte salt in capacitors.

8. Electrolyte containing one or more compounds of the general formula (I) as electrolyte salt and an electrolyte solvent.

9. Battery containing an electrolyte according to Claim 8.

10. Battery according to Claim 9 in which M⁺ in the formula (CF₃)₂CFSO₃-M⁺ (I) is Li⁺.

11. Capacitor containing a compound of the general formula (I) in which M⁺ is R₄P⁺ or R₄N⁺.

## Revendications

1. Composés de formule générale 1
(CF₃)₂CFSO₃⁻M⁺ (I)
où M⁺ représente K⁺, Na⁺, Li⁺, Cs⁺, Rb⁺, R₄P⁺ ou R₄N⁺, où les 4 groupes alkyle R peuvent être identiques ou différents et représentent C₁-C₄-alkyle ou au moins deux groupes R comprennent l'azote ou le phosphore et forment un système cyclique non aromatique.

2. Composés selon 1a revendication 1, où M⁺ représente Li⁺ ou R₄N⁺, où les 4 substituants R peuvent être identiques ou différents et signifient C₁-C₂-alkyle.

3. Procédé pour la préparation de composés de formule générale (I),
A) où on transforme, pour la préparation des sels de métal alcalin
a) (CF₃)₂CFSO₂F avec de l'hydroxyde de métal alcalin, de préférence en solution aqueuse et on sépare l'heptafluoropropanesulfonate de métal alcalin du fluorure de métal alcalin formé, ou
b) on transforme (CF₃)₂CFSO₂F avec Ca(OH)₂, de préférence en solution aqueuse, on sépare l'heptafluoropropanesulfonate de calcium formé du fluorure de calcium précipité, on transforme l'heptafluoropropanesulfonate de calcium avec un acide, de préférence l'acide sulfurique, en acide heptafluoropropanesulfonique et en sel calcique de l'acide et on transforme l'acide heptafluoropropanesulfonique avec un hydroxyde de métal alcalin, de préférence en solution aqueuse, en formant de l'heptafluoropropanesulfonate de métal alcalin, ou
B) où on transforme, pour la préparation des sels de tétraalkylammonium
a) l'halogénure de tétraalkylammonium avec un sel de métal alcalin de l'heptafluoropropanesulfonate et on isole l'heptafluoropropanesulfonate de tétraalkylammonium formé, ou
b) un hydroxyde de tétraalkylammonium avec de l'acide heptafluoropropanesulfonique, ou
C) où on transforme, pour la préparation des sels de tétraalkylammonium ou de tétraalkylphosphonium un alkylcarbonate de tétraalkylammonium ou de tétraalkylphosphonium avec de l'acide heptafluoropropanesulfonique.

4. Utilisation de composés de formule générale (I) dans l'électrotechnique.

5. Utilisation selon la revendication 4, de composés de formule générale (I), où M⁺ représente Na⁺, K⁺, Cs⁺, Rb⁺, ou Li⁺, comme sel conducteur pour batteries.

6. Utilisation selon la revendication 5, où M⁺ représente Li⁺, comme sel conducteur pour des batteries à ions de lithium.

7. Utilisation de composés de formule générale (I), où M⁺ représente R₄P⁺ ou R₄N⁺, comme sel conducteur dans des condensateurs.

8. Electrolyte, contenant un ou plusieurs composés de formule générale (I) comme sel conducteur et un solvant d'électrolyte.

9. Batterie, contenant un électrolyte de la revendication 8.

10. Batterie selon la revendication 9, où M⁺ dans la formule (CF₃)₂CFSO₃-M⁺ (I) signifie Li⁺.

11. Condensateur, contenant un composé de formule générale (I), où M⁺ représente R₄P⁺ ou R₄N⁺.
